# EUROPEAN PATENT SPECIFICATION

(11) **EP 2 910 257 B1**
(45) Date of publication and mention of the grant of the patent: **02.05.2018**
(21) Application number: 13848013.2
(22) Date of filing: 17.10.2013
(51) Int. Cl.: A61M 1/02, A61B 5/15, A61M 1/00

(54) **DEVICE FOR EXTRACTING BLOOD FROM THE PLACENTA AND THE UMBILICAL CORD**
VORRICHTUNG ZUR BLUTEXTRAKTION AUS DER PLAZENTA UND DER NABELSCHNUR
DISPOSITIF D'EXTRACTION DU SANG DU PLACENTA ET DU CORDON OMBILICAL

(30) Priority: 18.10.2012 ES 201231607
(43) Date of publication of application: 26.08.2015
(73) Proprietor: AGAR DESARROLLOS INNOVADORES EN BIOTECNOLOGIA, S.L., 28029 Madrid (ES)
(72) Inventor: ÁLVAREZ RAMOS, Ángel Gabriel, 33201 Gijón (ES)
(74) Representative: Manuel Illescas y Asociados, S.L.U.
(86) International application number: PCT/ES2013/070710
(87) International publication number: WO 2014/060626

(56) References cited:
- WO-A2-2005/041772
- ES-A1- 2 368 083
- JP-A- H07 184 991
- US-A- 5 059 168
- US-A- 5 372 581
- US-A- 5 915 384

## Description

### FIELD OF THE INVENTION

The invention relates to a device for extracting and collecting blood from the placenta and/or umbilical cord, more specifically to a device based on the collection of this blood by allowing it to fall under gravity in combination with a suction system enabling improvement of the collection process.

### BACKGROUND OF THE INVENTION

In recent years, the discovery of the existence of a high content of stem cells in the umbilical cord and in the placenta has encouraged the development of various systems of extracting and collecting blood contained in these organs in order to store it for subsequent use, either for scientific aims or, if required, to be transplanted into patients suffering from certain congenital or acquired diseases such as immunodeficiencies, cancers or metabolic disorders of deposition.

For transplantation of blood from the umbilical cord and the placenta to be effective, providing the required therapeutic level in the pathologies to be treated, it is crucially important to obtain a sufficient volume of blood rich in stem cells, requiring at least in the order of 1.5 x 10⁷ precursor cells per kg of patient body weight for a successful transplant. Currently, the vast majority of systems for blood extraction from the placenta and the umbilical cord are based on sterilised devices for puncturing the umbilical cord immediately after the expulsion of the neonate, during the minutes before the detachment of the placenta. The blood thus extracted falls under the effect of gravity into the blood collection bag, this bag being kept below the height of the woman who has just given birth, thereby encouraging the falling of the blood. Normally, the bag used includes anticoagulant substances and is kept in motion during the collection process, encouraging the preservation of the blood under optimal conditions.

Using the method described above, the average volume of blood collected in each delivery is estimated at approximately 120 ml. Although this volume is often sufficient to obtain a stem cell content that is later therapeutically effective, in a not insignificant number of patients this volume does not contain the stem cell content necessary as there are insufficient cell numbers.

Currently, the state of the art includes some systems for obtaining an increase in the blood extracted and collected from the placenta and the umbilical cord, mainly through pressure systems for aspirating this blood, thereby increasing the volume collected and reducing the collection time. Examples of these types of systems are the devices described in patents WO2005/041772, US5097842, US5059168 and US5575795. These devices include one or more needles for perforating the umbilical vein and/or placenta, means of collecting the blood such as reservoirs, bags or vacuum containers, and means of aspiration or drainage such as vacuum pumps or peristaltic pumps. In all the references cited above, increasing the amount of blood collected is achieved by replacing the usual method, based on a puncture and subsequent fall under gravity, by a method based exclusively on negative pressure exercised by means of suction or drainage. Japanese publication number JPH07184991 A (1995.07.25) discloses an umbilical blood taking appliance comprising: a juncture to the umbilical cord or the placenta, a manual liquid feeding means having suction and liquid feed functions, a blood taking vessel and a communicating means for connecting this liquid feeding means to the juncture and the blood taking vessel. This communicating member has a three-way stock cock having a selective communicating function to make the liquid feeding means selectively communicate with the juncture or the blood taking vessel. Although the devices are capable of carrying out the purposes for which they were designed, producing an increase in the amount of blood collected, they share a common problem related to their basic design being exclusively based on a suction method. As a consequence of the continuous application of this suction, there is the possibility of inducing the appearance of complications such as the sudden appearance of retroplacental haematoma and, consequently, premature detachment of the placenta before detachment naturally takes place and the maternal homeostasis factors at the time of childbirth can minimise bleeding. This detachment, which may be precipitated by a constant uncontrolled suction action of blood, involves an obvious health risk to the mother.

It is therefore necessary to develop devices that enable increasing the volume of blood collected from the placenta and the umbilical cord, so that the content of extracted stem cells is sufficient to meet the therapeutic needs and, at the same time, are safe for the mother during the time of delivery, ensuring natural detachment of the placenta without any risk to health.

The present invention aims to satisfy this need by a device for blood collection that combines the usual system of falling under gravity together with the controlled assistance supplied by a suction system.

Another advantage of the present invention is that with a design that combines extraction by falling under gravity and extraction by suction, as much blood can be extracted from the umbilical cord as that contained in the placenta by only puncturing the umbilical cord. Not needing to puncture the placenta is especially important as such a puncture normally implies a risk of bacterial contamination that can be avoided with the device described here. Additionally, the possibility of collecting blood from the placenta through a perforation of the umbilical cord makes it possible to carry out blood collection immediately after the expulsion of the neonate without needing to wait for the expulsion of the placenta, which can occur in a time from 5 to 30 minutes after the birth of the neonate, implying an optimisation in the working time of the surgeon and the consequent cost savings.

### BRIEF DESCRIPTION OF THE INVENTION

An object of the present invention is a device for blood collection from the placenta and the umbilical cord that combines collection of blood by falling under gravity and simultaneously a suction system that facilitates this falling.

This object is achieved by a first preferred embodiment of the invention consisting of a device for blood collection from the placenta and the umbilical cord comprising a means of blood extraction constituted by at least one needle or catheter and at least one tube or cannula through which the extracted blood circulates, this blood falling under the effect of gravity into a means of collection; and where the device also comprises a means of suction that is connected to the tube or cannula and is also connected to the means of blood collection through this tube or cannula. This provides an efficient way to increase the volume of blood collected from the placenta and/or the umbilical cord compared to the devices of the current state of the art, additionally offering a technical solution that enables controlling the pressure for suction of the blood, thus enabling an enormous reduction in the risks derived from excessive applied pressure, or from continuous pressure that is produced by the known devices for blood collection.

Preferably, the means of blood extraction include at least one needle or catheter and a tube or replacement cannula. In this way there is an exchangeable system directly coupled to the extraction device that can be used in case of need without having to replace any additional element that would imply a delay or complication in the collection process.

In another preferred embodiment of the present invention, the tube comprises at least one means of closure, for example through clamps or, alternatively, systems of by-pass valves operated either manually or automatically. This provides means of control and retention of the collected blood. In addition to the by-pass valve or clamp, the collection device can also comprise within the system of valves, for example, two- or more way shut-off valves and/or no-return or anti-return valves. This provides a control and retention system for collected blood that enables the optimum configuration of the elements of the device in its collection circuit so that the volume of blood obtained can be maximised, additionally ensuring that the collected blood remains inside the device, without returning to the umbilical cord or to the mother. The point of connection of the means of suction to the tube or cannula is located at a distance of less than 50 % of the maximum length of this tube or cannula from the collection device or bag, being preferably located less than 30 % of the maximum length of the cannula from this bag. In this way, a suitable distance is achieved to ensure both a substantial increase in blood collected and the realisation of a gentle pressure not applied too close to the umbilical cord (which could cause undesirable excessive suction). However, depending on the specific features of the type of blood collection that is being carried out, the position of both the means of suction and the optional means of closure can vary, they being located, for example close to the means of collection. In a preferred embodiment of the present invention, the means of collection contains anticoagulants. These preserve the collected blood under optimal conditions.

In a preferred embodiment of the present invention, the means of suction can be operated manually or automatically, preferably by a manually operated syringe. This makes the device adaptable to different functions, depending on the requirements of each collection process.

In a preferred embodiment of the invention, at least the collection device or bag is sterile and, in a more preferred embodiment, the whole of the collection device with all its components is sterile: needle(s), cannula(s), bag(s), valve(s), means of suction.

Other characteristics and advantages of the present invention will emerge from the description of the invention that follows, as well as from the embodiment illustrated in the accompanying figure.

### DESCRIPTION OF THE FIGURES

Figure 1 shows schematically the first preferred embodiment of the device described in the present invention.
Figure 2 shows schematically the second preferred embodiment of the device described in the present invention.

### DETAILED DESCRIPTION OF THE INVENTION

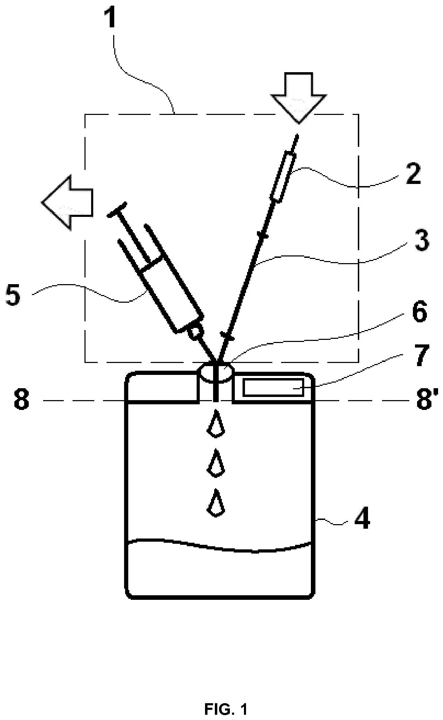

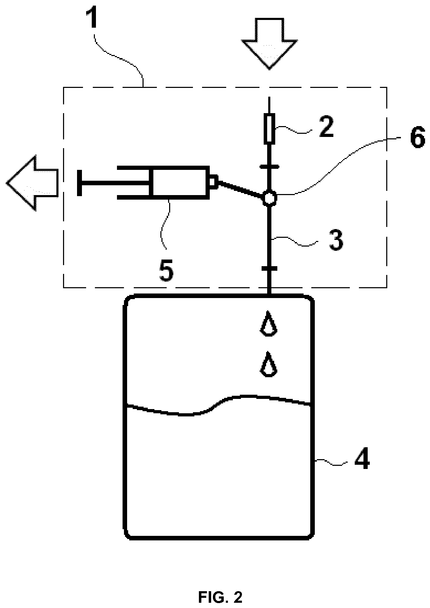

In accordance with the invention and according to the embodiments shown in Figures 1 and 2, the device for blood extraction and collection is constituted by at least one means of blood extraction (1) that preferably comprises at least one needle or catheter (2) for puncturing the umbilical vein and/or venous system of the placenta and at least one tube or cannula (3) through which the extracted blood flows. In different embodiments of the present invention it is possible to have more than one tube and needle as a replacement system in case of need. Each tube (3) is connected to at least one means of blood collection (4) that preferably comprises a collection bag, preferably incorporating anticoagulants for preserving the blood. Preferably, this bag is not subjected to prior vacuum.

The device of the present invention also comprises a means of suction (5) that is preferably constituted by a plunger system, operated manually or automatically. This means of suction is connected to the tube (3) and to the means of collection (4) through this tube (3).

Preferably, the tube (3) comprises at least one means of closure (6), for example by clamps or, alternatively, a by-pass valve system, operated manually or automatically. Additionally or alternatively to the by-pass valves, the collection device of the invention can also comprise closure valves of two- or more ways and/or non-return valves, for example, within the valve system. In this way, it is possible to have a device capable of exercising a negative pressure on the blood of the umbilical cord that carries the blood towards the means of suction (5) and that at the same time ensures that the collected blood is later kept inside the device, when the blood is transferred from this means of suction (5) towards the means of collection (4) by applying a positive pressure, reversing the movement of the plunger. In order to encourage non-return of the aspirated blood towards the umbilical cord, it is also possible to apply the means of suction (5) oriented substantially towards the means of collection (4) by, for example, an application route in the form of a "V", arranged in the upper part of the means of collection (4) at the point of entry of this tube or cannula (3) into the means of collection (4) (see Figure 1).

In an embodiment of the invention, the device or collection bag has in its upper part a zone (7) not to be filled with blood, arranged to house the entry cannula in the form of a "V", coupled to the means of suction (5) and, eventually, with a two- or more way closure or by-pass valve. This zone (7) can be of the same material or a different material from the rest of the device or bag for blood collection. It is preferably made of a material that will allow writing on it the data about the collection, identity of the patient, etc. In this preferred embodiment of the invention, the mentioned zone (7) can allow the sticking of labels with the information referred to above, or can even have them already stuck or printed.

The point of connection of the means of suction (5) to the tube (3) is located at a distance of less than 50 % of the maximum length of this tube (3) from the means of collection (4) or bag, being preferably located less than 30 % of the maximum length of the tube (3) from said bag, so that a substantial increase in the collected blood is obtained and a gentle pressure can be applied not too close to the umbilical cord. In different embodiments of the invention, depending on the specific characteristics of the type of blood collection being carried out, the position both of the means of suction (5) and of the optional means of closure (6) can vary, they being located, for example, close to the needle, close to the means of collection, in an intermediate region, or in a combination of configurations of these positions of these means (5, 6).

In the various embodiments of the invention, the blood collection device is preferably sterilized.

The blood collection procedure using the device of the present invention thus comprises the following stages:
a) The umbilical vein and/or venous system of the placenta is punctured with a needle (2). Optionally, the umbilical vein can be punctured to extract the blood contained in the placenta without needing to puncture the latter.
b) The means of collection (4) is kept at a height below that of the mother and in constant motion encouraging respectively, the falling of blood under gravity and the preservation of the blood under optimal conditions.
c) The means of suction (5) is used to help the extraction of blood and its falling under gravity through the tube (3) towards said means of suction (5) in a controlled way, applying a sufficiently gentle negative pressure to prevent the risk of detachment of the placenta, the amount of this pressure being estimated at the discretion of the physician, depending on the characteristics of each patient and the circumstances of the delivery.
d) After carrying out the collection of a volume of blood towards the means of suction (5), for example until the volume of this means of suction (5) is filled, the pressure is reversed by the operation of the plunger, transferring the volume of blood towards the means of collection (4), where it is kept together with the rest of the volume of blood collected during the entire operation. Optionally, to carry out this step, it is possible to apply means of closure (6) to control the flow of blood from the means of suction (5) to the means of collection (4). These means of closure (6) can be any of those on the market and comprise any of the systems of valves described in the present invention: clamp, by-pass, many-way (at least two-way) valves, non-return valves, etc. The means of closure (6) of Figure 1 can also consist of sealing off by simple folding / unfolding over of the means of collection or bag (4) in an embodiment of the invention in which the means of suction (5) is coupled in a "V" to the tube or cannula (3), in the region of its entry to the means of blood collection (4). The folding over of the means of collection (4) is preferably carried out along the line 8 - 8' in Figure 1.

By the process described above, it is possible therefore to draw residual blood in the placenta and the umbilical cord that is not accessible by a method based exclusively on falling under gravity. This enables a considerable increase in the volume of blood collected, as shown in the data of Table 1, where the results obtained in a sample of collections carried out with gravity collection bags are compared against the data obtained using the present device. Using the data in this table, it is possible to estimate the average increase in volume to be 51 ml when using the device for blood collection based on gravity plus suction of the present invention compared to the volume collected by the usual methods based on devices of gravity collection only. The best results were obtained with the second preferred embodiment of the invention of Figure 2 (means of suction (5) coupled to the tube or cannula (3), at a short distance, but outside the blood collection device or bag (4)), that showed an average increase in the volume of blood collected over the first preferred embodiment of the invention of Figure 1 (where the means of suction is coupled in a "V" to the tube or cannula (3) in the zone of entry to the means of collection (4)) of at least 10 %.

**Table 1**

| **Group** | **Collection carried out under gravity alone** | **Collection carried out with the present invention** |
|---|---|---|
| Samples | 9749 | 10 |
| Mean (ml) | 120.1 | 158 |
| Standard deviation (ml) | 28.96 | 46.5 |
| Variance (ml) | 838.8 | 1946.6 |
| Median (ml) | **117** | **168** |
| 95 % confidence interval (ml) | 119.5 - 121 | 125.5- 192.1 |

Furthermore, the existence of a means of suction in the device of the invention, that can be operated in a controlled way and designed to support the method of blood extraction and collection under gravity, is able to provide the guarantee that premature detachment of the placenta will not take place, so preventing the risks to the health of the mother that accompany the methods of collection by suction of the current state of the art.

All the embodiments described for the present invention should not be considered as limiting of other variations in the design or the materials employed in manufacture, provided that these variations do not alter the essence of the invention and the object thereof.

## Claims

1. Device for blood collection from the placenta and/or the umbilical cord comprising at least one means of blood extraction (1) constituted by at least one needle or catheter (2) and, at least, one tube or cannula (3) through which the extracted blood flows, where the tube or cannula (3) is connected to, at least, one means of blood collection (4), this blood being extracted by falling under gravity into the means of collection (4) and this device being **characterised in that** it also comprises a means of suction (5) of blood, used to help in the extraction of blood, said means of suction (5) of blood is connected to the tube or cannula (3) and also connected to the means of blood collection (4) by this tube or cannula (3); and **in that** the point of connection of the means of suction (5) to the tube or cannula (3) is located at a distance of less than 50% of the maximum length of the cannula from the means of collection (4).

2. Device for blood collection according claim 1, wherein the means of blood extraction (1) additionally comprise at least one replacement needle or catheter (2) and tube or cannula (3).

3. Device for blood collection according to any of claims 1 - 2, wherein the tube or cannula (3) comprises at least one means of closure (6) of said tube or cannula (3).

4. Device for blood collection according to the previous claim, wherein the means of closure (6) of the tube or cannula (3) comprises one or more clamps.

5. Device for blood collection according to any of claims 3 - 4, wherein the means of closure (6) of the tube or cannula (3) comprises a system of by-pass valves (6) operated manually or automatically.

6. Device for blood collection according to claim 3, wherein the means of closure (6) of the tube or cannula (3) comprises one or more non-return valves.

7. Device for blood collection according to claim 5, wherein the system of by-pass valves (6) comprises at least one two- or more way valves.

8. Device for blood collection according to the previous claim, wherein the point of connection of the means of suction (5) to the tube or cannula (3) is located at a distance of less than 30 % of the maximum length of the tube or cannula (4) from the means of collection (4).

9. Device for blood collection according to any of claims 1 - 8, wherein the means of suction (5) is connected to the tube or cannula (3) in a direction substantially orientated towards the means of collection (4), by an application route in the form of a "V" arranged in the upper part of the means of collection (4), at the point of entry of said tube or cannula (3) into said means of collection (4).

10. Device for blood collection according to any of claims 1 - 9, **characterised in that** the means of collection (4) contains anticoagulants.

11. Device for blood collection according to any of claims 1 - 10 **characterised in that** the means of suction (5) can be operated either manually or automatically.

12. Device for blood collection according to claim 11, wherein the means of suction (5) is a manually operated syringe.

13. Device for blood collection according to claim 9 comprising a means of closure (6) of the tube or cannula (3) which comprise the folding / unfolding over of the means of collection (4) in the region of entry of the means of suction (5) and the tube or cannula (3) into the means of blood collection (4).

14. Device for blood collection according to any of claims 9 or 13 comprising a zone (7) arranged to house the entry cannula in a "V", this zone (7) being configured to additionally allow writing information or sticking information labels.

## Patentansprüche

1. Vorrichtung zum Sammeln von Blut aus der Plazenta und/oder der Nabelschnur, umfassend mindestens ein Blutentnahmemittel (1), das aus mindestens einer Nadel oder einem Katheter (2) und mindestens einem Röhrchen oder einer Kanüle (3), wodurch das entnommene Blut fließt, besteht, wobei das Röhrchen oder die Kanüle (3) an mindestens ein Blutsammelmittel (4) angeschlossen ist und dieses Blut entnommen wird, indem es durch die Schwerkraft in das Sammelmittel (4) fällt, und diese Vorrichtung **dadurch gekennzeichnet ist, dass** sie weiterhin ein Saugmittel (5) für Blut umfasst, das für die Blutentnahme zu Hilfe genommen wird, wobei dieses Saugmittel (5) für Blut an das Röhrchen oder die Kanüle (3) angeschlossen ist und über dieses Röhrchen oder diese Kanüle (3) ebenfalls an das Blutsammelmittel (4) angeschlossen ist; und dass die Anschlussstelle des Saugmittels (5) an das Röhrchen oder die Kanüle (3) sich in einem Abstand vom Sammelmittel (4) befindet, der weniger als 50 % der maximalen Länge der Kanüle beträgt.

2. Vorrichtung zum Sammeln von Blut nach Anspruch 1, wobei das Blutentnahmemittel (1) zusätzlich mindestens eine Ersatznadel oder einen Ersatzkatheter (2) sowie ein Ersatzröhrchen oder eine Ersatzkanüle (3) umfasst.

3. Vorrichtung zum Sammeln von Blut nach einem der Ansprüche 1 bis 2, wobei das Röhrchen oder die Kanüle (3) mindestens ein Schließmittel (6) für dieses Röhrchen oder diese Kanüle (3) umfasst.

4. Vorrichtung zum Sammeln von Blut nach dem vorstehenden Anspruch, wobei das Schließmittel (6) für das Röhrchen oder die Kanüle (3) eine oder mehrere Klemmen umfasst.

5. Vorrichtung zum Sammeln von Blut nach einem der Ansprüche 3 bis 4, wobei das Schließmittel (6) für das Röhrchen oder die Kanüle (3) ein hand- oder automatisch betätigtes System aus Bypass-Ventilen (6) umfasst.

6. Vorrichtung zum Sammeln von Blut nach Anspruch 3, wobei das Schließmittel (6) für das Röhrchen oder die Kanüle (3) ein oder mehrere Rückschlagventile umfasst.

7. Vorrichtung zum Sammeln von Blut nach Anspruch 5, wobei das System aus Bypass-Ventilen (6) mindestens ein Zweiwege- oder Mehrwegeventil umfasst.

8. Vorrichtung zum Sammeln von Blut nach dem vorstehenden Anspruch, wobei die Anschlussstelle des Saugmittels (5) an das Röhrchen oder die Kanüle (3) sich in einem Abstand vom Sammelmittel (4) befindet, der weniger als 30 % der maximalen Länge des Röhrchens oder der Kanüle (4) beträgt.

9. Vorrichtung zum Sammeln von Blut nach einem der Ansprüche 1 bis 8, wobei das Saugmittel (5) im Wesentlichen in Richtung des Sammelmittels (4) über eine V-förmige, am oberen Bereich des Sammelmittels (4) angeordnete Anwendungsstrecke an der Zugangsstelle des Röhrchens oder der Kanüle (3) zu diesem Sammelmittel (4) an das Röhrchen oder die Kanüle (3) angeschlossen ist.

10. Vorrichtung zum Sammeln von Blut nach einem der Ansprüche 1 bis 9, **dadurch gekennzeichnet, dass** das Sammelmittel (4) Gerinnungshemmer enthält.

11. Vorrichtung zum Sammeln von Blut nach einem der Ansprüche 1 bis 10, **dadurch gekennzeichnet, dass** das Saugmittel (5) entweder hand- oder automatisch betätigt werden kann.

12. Vorrichtung zum Sammeln von Blut nach Anspruch 11, wobei das Saugmittel (5) eine handbetätigte Spritze ist.

13. Vorrichtung zum Sammeln von Blut nach Anspruch 9, die ein Schließmittel (6) für das Röhrchen oder die Kanüle (3) umfasst, das das Umknicken des Sammelmittels (4) im Zugangsbereich des Saugmittels (5) und des Röhrchens oder der Kanüle (3) zum Blutsammelmittel (4) umfasst.

14. Vorrichtung zum Sammeln von Blut nach einem der Ansprüche 9 oder 13, die einen zur Aufnahme des V-förmigen Kanülenzugangs angeordneten Bereich (7) umfasst, wobei dieser Bereich ausgebildet ist, um zusätzlich Informationen aufzuschreiben oder Informationsetiketten aufzukleben.

## Revendications

1. Dispositif pour prélèvement de sang provenant du placenta et/ou du cordon ombilical comprenant au moins un moyen d'extraction de sang (1) constitué par au moins une aiguille ou un cathéter (2) et, au moins, un tube ou une canule (3) à travers laquelle coule le sang extrait, où le tube ou la canule (3) est raccordée à, au moins, un moyen de prélèvement de sang (4), ce sang étant extrait en tombant par gravité dans le moyen de prélèvement (4) et ce dispositif étant **caractérisé en ce qu'**il comprend également un moyen d'aspiration (5) de sang, utilisé pour aider à l'extraction de sang, ledit moyen d'aspiration (5) de sang est raccordé au tube ou à la canule (3) et il est aussi raccordé au moyen de prélèvement de sang (4) par ce tube ou cette canule (3) ; et **en ce que** le point de raccordement du moyen d'aspiration (5) au tube ou à la canule (3) se situe à une distance inférieure à 50% de la longueur maximale de la canule à partir du moyen de prélèvement (4).

2. Dispositif pour prélèvement de sang selon la revendication 1, dans lequel le moyen d'extraction de sang (1) comprend en outre au moins une aiguille ou un cathéter (2) et un tube ou une canule (3) de rechange.

3. Dispositif pour prélèvement de sang selon l'une quelconque des revendications 1 - 2, dans lequel le tube ou la canule (3) comprend au moins un moyen de fermeture (6) dudit tube ou canule (3).

4. Dispositif pour prélèvement de sang selon la revendication précédente, dans lequel le moyen de fermeture (6) du tube ou de la canule (3) comprend un ou plusieurs clamps.

5. Dispositif pour prélèvement de sang selon l'une quelconque des revendications 3 - 4, dans lequel le moyen de fermeture (6) du tube ou de la canule (3) comprend un système de vannes de dérivation (6) actionné manuellement ou automatiquement.

6. Dispositif pour prélèvement de sang selon la revendication 3, dans lequel le moyen de fermeture (6) du tube ou de la canule (3) comprend un ou plusieurs clapets de non-retour.

7. Dispositif pour prélèvement de sang selon la revendication 5, dans lequel le système de vannes de dérivation (6) comprend au moins une vanne à deux ou plusieurs voies.

8. Dispositif pour prélèvement de sang selon la revendication précédente, dans lequel le point de raccordement du moyen d'aspiration (5) au tube ou à la canule (3) se situe à une distance inférieure à 30% de la longueur maximale du tube ou de la canule (4) à partir du moyen de prélèvement (4).

9. Dispositif pour prélèvement de sang selon l'une quelconque des revendications 1 - 8, dans lequel le moyen d'aspiration (5) est raccordé au tube ou à la canule (3) dans un sens substantiellement orienté vers le moyen de prélèvement (4), par une voie d'utilisation en forme de V disposée dans la partie supérieure du moyen de prélèvement (4), au point d'entrée dudit tube ou de ladite canule (3) dans ledit moyen de prélèvement (4).

10. Dispositif pour prélèvement de sang selon l'une quelconque des revendications 1 - 9, **caractérisé en ce que** le moyen de prélèvement (4) contient des anticoagulants.

11. Dispositif pour prélèvement de sang selon l'une quelconque des revendications 1 - 10, **caractérisé en ce que** le moyen d'aspiration (5) peut être actionné soit manuellement soit automatiquement.

12. Dispositif pour prélèvement de sang selon la revendication 11, dans lequel le moyen d'aspiration (5) est une seringue actionnée manuellement.

13. Dispositif pour prélèvement de sang selon la revendication 9 comprenant un moyen de fermeture (6) du tube ou de la canule (3) qui comprend le pliage /dépliage au-dessus du moyen de prélèvement (4) dans la région d'entrée du moyen d'aspiration (5) et du tube ou de la canule (3) dans le moyen de prélèvement de sang (4).

14. Dispositif pour prélèvement de sang selon l'une quelconque des revendications 9 ou 13 comprenant une zone (7) disposée pour loger la canule d'entrée en V, cette zone (7) étant configurée pour permettre en outre d'écrire des informations ou de coller des étiquettes d'information.
